# EUROPEAN PATENT SPECIFICATION

(11) **EP 3 021 797 B1**
(45) Date of publication and mention of the grant of the patent: **12.04.2023**
(21) Application number: 14732126.9
(22) Date of filing: 12.06.2014
(51) Int. Cl.: A61F 2/915, A61F 2/88

(54) **STENT**
STENT
ENDOPROTHÈSE

(30) Priority: 19.07.2013 US 201361856073 P; 19.07.2013 EP 13177182
(43) Date of publication of application: 25.05.2016
(73) Proprietor: Biotronik AG, 8180 Bülach (CH)
(72) Inventor: BAKCZEWITZ, Frank, 18055 Rostock (DE); PÖHLMANN, Stefanie, 95466 Weidenberg (DE); AMIDO, Alexandre, 18057 Rostock (DE)
(74) Representative: Biotronik Corporate Services SE
(86) International application number: PCT/EP2014/062190
(87) International publication number: WO 2015/007435

(56) References cited:
- EP-A1- 1 516 600
- EP-B1- 1 430 854
- WO-A1-00/62710
- US-A- 5 810 872
- US-A1- 2004 106 985
- US-A1- 2009 163 991
- US-A1- 2011 137 407

## Description

The invention relates to a stent comprising a preferably cylindrical circumferential wall designed as an open, lattice-like, expandable supporting structure with a longitudinal axis, having the features specified in the preamble of claim 1.

A stent of this type is known for example from EP 1 430 854 B1 and has helix webs running around helically in windings about the longitudinal axis with a helical primary shape and a meandering secondary shape. The latter forms meander bends with alternating direction of curvature in the circumferential direction and also forms sub-webs connecting the meander bends. The helix webs of two adjacent windings are bridged in this case by connecting webs with a primary direction of extension parallel to the longitudinal axis of the circumferential wall, said connection webs being attached at their ends facing towards the sub-webs to adjacent sub-webs of the corresponding helix web and have a flat, zigzag-like shape with two end branches and an intermediate central branch.

Another type of stent with a lattice-like expandable supporting structure is known from EP 2 438 872 A1 and is formed by a supporting structure lattice constructed from diamond-shaped grid cells. The bends in the region of the acute-angled intersections of the respective sub-webs of this diamond-shaped grid structure are provided with recesses for producing an inner radius in these regions.

US 2011/0137407 discloses an implantable medical devices may be utilized to locally delivery one or more drugs or therapeutic agents to treat a wide variety of conditions, including the treatment of the biological organism's reaction to the introduction of the implantable medical device. These therapeutic agents may be released under controlled and directional conditions from a stent so that the one or more therapeutic agents reach the correct target area, for example, the surrounding tissue.

US 2009/0163991 discloses a stent with strut bands and connectors, wherein the strut bands have long and short struts with a junction positioned between the short struts. Each junction defines a reservoir, wherein the reservoirs of a strut band are substantially circumferentially aligned. The connectors each have arms, wherein each arm includes an opposing U-shaped link. The opposing links have a shared portion disposed between a peak on one strut band and a longitudinally adjacent trough of an adjacent strut band.

WO 00/62710 discloses a medical device includes a catheter and a stent mounted on the catheter, the stent having a hollow, cylindrical body made with a plurality of rings. The rings each extend circumferentially around the cylindrical body and include an undulating series of peaks and valleys. The rings are joined together by a series of links which are shaped and arranged to promote longitudinal flexibility as the stent is delivered on the catheter and effective scaffolding after deployment. In one embodiment, the rings are provided with inflection points on some portions of the rings which extend in a generally circumferential direction for a short distance. A link is joined at one end at the inflection point on one ring and also joined at a second end at a second inflection point on an adjacent ring. This construction allows the crimped stent to flex longitudinally when it is subjected to bending forces such as those encountered during delivery of the stent and catheter through a tortuous coronary artery.

US 2004/0106985 discloses a stent comprises a first expansion strut column of first expansion strut pairs and a second expansion strut column of second expansion strut pairs. Each first expansion strut pair is connected to a second expansion strut pair by a connecting strut. Each connecting strut comprises at least one wrap portion, the at least one wrap portion being at least partially wrapped about at least one of the first joining portions of at least one of the first expansion strut column and the second expansion strut column.

EP 1516600 discloses a stent with struts and bends forming a helical structure. The turns of the helix are connected by undulating struts which extend from a bend of one helical turn to a bend of the adjacent turn.

US 5810872 discloses a stent with struts and bends forming a helical structure. The turns of the helix are connected by straight struts which extend from a bend of one helical turn to a bend of the adjacent turn.

In relation to the background of the invention, the function of a stent is to be recapitulated, said stent being intended to support the wall of a bodily vessel in a suitable manner, to expand the vessel, or to bridge an aneurysm for example. To this end, stents are introduced into the vessel in a compressed state, are expanded at the site to be treated, and are pressed against the vessel wall. This expansion can be achieved with the aid of a balloon catheter for example, however self-expanding stents are also known alternatively.

In principle, the desired properties of a stent are manifold. Basic requirements are as simple expandability of the stent as possible with simultaneously simple positionability. In the case of balloon-expandable stents, it is also desirable for the stent to spring back resiliently to a minimal extent after the expansion. Lastly, stents must have sufficient bearing capacity, but also a specific flexibility about their longitudinal axis in order to participate in conventional vessel movements.

A broad range of various designs and microstructurings of the lattice-like supporting structure consistently target an optimal design of the stent with regard to the respective purpose and the primarily desired property features.

In the present case, a homogeneous covering of the vessel wall with avoidance of significant jumps in radial stiffness in the lateral surface supporting the vessel wall is ensured by the helical structure of the stent design. Proceeding here from, the object of the invention is to ensure a small crimp profile, that is to say an embodiment of the stent that is as compact as possible in the contracted state, and also a high bending flexibility. In the case of balloon-expandable stents, this is to be accompanied by a low opening pressure.

This object is achieved by the design, as specified in the characterizing part of claim 1, of at least the end branch of the connection webs bridging the helix windings. The end branches each lead via a termination bend at least approximately at right angles into the respective sub-web and the corners in the mouth region between the termination bends and the sub-webs are provided with a recess.

All specified diameters, orientations, angles, lengths, radii or other dimensional parameters refer to the stent in the state before crimping, except explicitly stated otherwise. The state of the stent before crimping onto a catheter is the state in which the production of the stent is concluded. In this state, it is applied ("crimped") onto the respective catheter in order to be introduced into the bodily vessel. There, it is then expanded to the respective diameter of the bodily vessel. The form in the expanded state, in particular the alignment of the individual webs and bends, then corresponds substantially to the state before crimping, wherein modifications due to the expansion and the adaptation to the bodily vessel are possible however.

In the state before crimping, the stent is independent of the used catheter and independent of the body vessel to be implanted. Sometimes this state is referred as "cut out" or manufactured state or shortly within this application as the unexpanded state.

Within the frame of this application, the mouth region is understood as part of the sub-web where the sub-web and the termination bend of the connector are joined. The center of the mouth region is the intercept between the center of gravity line of the termination bend and the center of gravity line of the sub-web. The width of the mouth region is between four and six times the width of the termination bend.

The combination of the right angle and the recesses in the mouth region leads to a surprisingly low crimping profile. A skilled person might expect a rectangular connection between the termination bends and the sub-webs would increase the crimping diameter. Surprisingly the combination with the recess leads to a lower possible crimping profile. Additionally the combination of the right angle and the recess improves the axial flexibility of the stent crimped on the catheter which improves the pushability of the stent-catheter system through tortuous body vessels.

Each of the end branches of the connection webs leads at least approximately at right angles via a termination bend into the respective sub-web. Here, the respective termination bend and the deflection bend arranged therebefore of each connection branch is curved in the opposite direction. This microshaping also in turn optimizes the crimpability of the stent for transfer into this contracted state.

The termination bends of the end branches of the connection webs inherently form with the sub-webs in the mouth region an approximately right-angled corner, which is provided with a recess, which not only rounds out said mouth region, but also thins the meander segment. Due to the recess, the width of the sub-webs in the region of the recess is reduced preferably by 5 % to 50 %. In particular, the width is reduced preferably by 20 % to 30 %, particularly preferably by 24 % to 28 %. Due to this arcuate recess and the associated simplified horizontal placement of the connectors, a lower required opening force of the stent in order to transfer from the contracted state into the expanded state is ensured. Due to this recess and the inner radius created thereby, slit-shaped undercuts in the mouth region, which could lead to the problems already mentioned above during a surface-finishing process, are also avoided.

Further preferred developments of the subject of the invention are specified in the dependent claims.

The end branches have inherently a progression with at least one deflection bend which is flat in such a way that the radius of said deflection bend corresponds to 1 to 15 times the radius of an adjacent meander bend and the inner face of said deflection bend is arranged adjacently to the outer face of said meander bend of the secondary shape of the helix webs in the unexpanded state of the stent. Due to this "microshaping", the connection webs can nestle optimally between the meander bends during the crimping process, that is to say as the stent is transferred into its contracted state, for insertion into the vessel. As the stent expands, the connection webs are aligned in their primary direction of extension parallel to the longitudinal axis of the stent and thus improve the structural stability of the stent *per se* and therefore also ensure a homogeneous distribution of the supporting metal elements of the stent.

Within the scope of the invention, the center of gravity lines of the deflection bend and of the meander bend are used in order to determine the ratio of the radii of the deflection bend and of the meander bend. The center of gravity line connects the midpoint of the respective cross-sectional areas of a deflection bend or a meander bend. The center of gravity line is therefore accordingly a curved line having the same center as the deflection bend or the meander bend.

Here, within the scope of the application, "flat" is understood to mean that a flat wave has a ratio of wavelength to amplitude of at least 13:1, preferably at least 20:1.

Furthermore, within the scope of this application, the phrase "inner face" or respectively "outer face" is defined with respect to the curve of the respective bends. The side or face of the bend closer to the origin or center point of the curve is referred as "inner face". The opposite side of the bend is referred as "outer face".

Here, within the scope of the application a "deflection bend" is understood as web in form of a bend, which is oriented in a direction around another structure to avoid contact with another structure.

Due to the shaping according to this preferred embodiment with flat deflection bends in the region of the end branch and preferably also the central branch of the connection webs, a sufficient distance from the adjacent portion of the meandering secondary shape of the helix webs is also achieved, such that a long, narrow undercut region is avoided, which may lead to process problems when finishing the surface of the stent, for example by means of electropolishing and coating.

In spite of the attachment of the connection webs from web center to web center of the sub-webs of the helix webs, a compact crimp profile in the contracted state of the stent is also achieved by the specifically recessed connection webs, which nestle optimally between the meander bends of the helix webs during the crimping process.

In a preferred embodiment of the invention, the radius of the flat deflection bend corresponds to 1.3 to 12 times, preferably four to 11 times, particularly preferably six to 8 times, the radius of an adjacent meander bend.

In particular, the radius of the flat deflection bend is preferably 1.2 to 5 times or 6 to 8 times the radius of the adjacent meander bend.

Due to the design of the central branch as an undulating deflection bend, an optimal adaptation of the connection web to the meander bends, arranged on either side thereof, of two adjacent helix webs is thus possible. The central branch is designed in particular as a flat undulating deflection bend.

Here, within the scope of the application, an undulating deflection band is understood as a deflection band in the sense as mentioned above, which is undulated or corrugated.

In accordance with a preferred embodiment of the invention, the radius of the compensation bend of the central branch also corresponds to 1 to 15 times the radius of the adjacent meander bend. For this embodiment, similar ratios for the radii as disclosed previously are preferred. The ratio of the radii of the deflection bends of the end branch and/or of the central branch to the radius of the adjacent meander bend meets the previously disclosed conditions, wherein the ratio of the radii for the deflection bends of the end branch and of the central branch can each be different.

In preferred embodiments of the invention, the ratio of the radius of the deflection bend of the end branch to the radius of the adjacent meander bend is 11, and the ratio of the radius of the deflection bend of the central branch to the radius of the adjacent meander bend is 2.3. Other expedient combinations of the ratios are, for example, 4.7 and 11, and 1.3 and 10.7, and also 2.6 and 6.2.

In the embodiments of the invention where both the radius of the deflection bend of the end branch and also the radius of the deflection bend of the central branch meets the above-mentioned conditions, the advantages of the invention are particularly effective. The connectors nestle optimally against the adjacent meander bends, whereby a small and compact state when crimping onto the balloon is achieved.

Since the deflection bends of the end branch and central branch merely have to imitate the outer radius of the meander bends arranged adjacently thereto, a comparatively low bend angle, which is preferably 45° at most, is sufficient besides the relatively high radius of curvature, as is provided in accordance with the invention, which for example may correspond at least to three times the radius of curvature of the meander bend. The optimal shape for nestling the connection branch between the meander bends arranged adjacently thereto is thus found. The bend angle of the deflection bends of the end and/or central branch is preferably between 4° and 30°. Here, the bend angle of the deflection bends of the end branch and of the central branch within the specified range may advantageously have a difference of up to 20°, preferably up to 15°, particularly preferably up to 10°.

For the formation of the cylindrical circumferential wall, three helix webs are preferably provided, running around helically about the longitudinal axis of the stent in the manner of a thread having three turns. Due to this basic construction in the primary form of the supporting structure, a very homogeneous distribution of stiffness of the supporting structure is obtained in the longitudinal direction of the stent. In order to form the pitch of the helix webs, an asymmetrical meander structure can be provided here, in which each second sub-web of a helix web following successively in the circumferential direction has a greater length than the sub-webs arranged therebetween. The pitch of the helix webs can preferably be set by means of this difference in length. Further parameters for the pitch also include however the angle between the webs and the general angular alignment thereof relative to the longitudinal axis of the stent. With a formation of the helix structure with more than three turns, the difference in length is likewise greater than with an embodiment having three or two turns.

To further optimize the effect of the reciprocal nestling of the different webs of the supporting structure, the longer sub-webs of the helix webs can be curved in a flat S-shaped manner.

In accordance with a preferred development of the invention, the connection webs of two helix webs following successively in pairs are arranged offset in relation to the connection webs of the adjacent pairs of helix webs, in such a way that one connection web at most leads into a sub-web of the helix webs. Due to this design, no two connectors meet at a sub-web, and the mechanical load of the supporting structure during expansion is therefore distributed more uniformly.

Further preferred embodiments concern the termination of the supporting structure at the longitudinal ends of the cylindrical circumferential wall, where there is the problem that the helix webs of the primary form running in diagonally to the end of the stent have to be terminated in an "orderly" manner. On the one hand, this is optimized by arranging a peripheral edge segment with symmetrical meander structure at the two longitudinal ends of the circumferential wall. The transition of the helix webs can then be implemented with increasing reduction of the length of their sub-webs in this edge segment. Alternatively, in accordance with a preferred embodiment, a transition segment with adapted radii of the meander bends and suitably dimensioned lengths of the sub-webs is to be formed between the edge segment and the helix webs, wherein the helix webs are then discontinued in this transition segment without reduction of the length of their sub-webs.

In accordance with a further preferred formation of the micro structuring of the helix webs, a widening of the sub-webs is provided between the meander bends and is caused by flat, outwardly protruding ramps at the transition region between meander bend and sub-web. The expansion behavior and the flexibility of the supporting structure are thus improved, and the stress-strain state of said supporting structure is optimized.

To summarize, a large number of advantages are provided by the embodiment of the stent according to the invention:
- Due to the specific design of the connection webs and variable web dimensioning, the stent itself has high flexibility.
- Due to the at least triple helix, suitably shortened segments and therefore more meander bends over the circumference of the stent supporting structure provide an improved adaptability of the stent to the different purposes.
- Due to the design according to the invention, in particular if the meander bends form a triple helix structure, the stent can nestle particularly well against the vessel wall of the surrounding bodily vessel. This is advantageous in particular if the stent according to the invention is coated with a therapeutic active ingredient, since the active ingredient can thus enter the bodily vessel effectively.
- The stent achieves a high radial force and a low opening pressure.
- On the whole, the stent design ensures an optimized stress-strain state and considerable resistance to signs of fatigue caused by permanent dynamic load in clinical use.

Further features, details and advantages of the invention will emerge from the following description of exemplary embodiments on the basis of the accompanying drawings, in which:
- Figure 1: shows a detail of a development of a lattice-like supporting structure of a stent in the state before crimping onto a catheter,
- Figure 2: shows the detail II according to figure 1 in the state before crimping onto a catheter,
- Figure 3: shows a detail similar to figure 2 in the contracted state of the supporting structure,
- Figure 4: shows a plan view, again enlarged, of a detail of a helix web in the state before crimping onto a catheter,
- Figure 5: shows a view similarly to figure 1 of a supporting structure in an alternative embodiment of one of its longitudinal ends in the state before crimping onto a catheter,
- Figure 6: shows a connection web of an alternative embodiment of the invention in the state before crimping onto a catheter, and
- Figure 7: shows a connection web of a further alternative embodiment of the invention in the state before crimping onto a catheter.

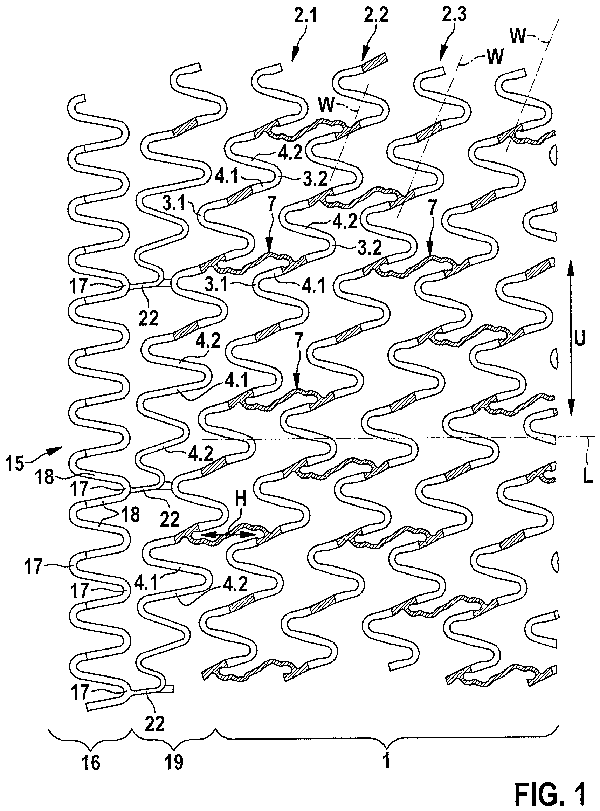

In figure 1, a detail of the cylindrical circumferential wall 1 of a stent designed as an open, lattice-like, expandable supporting structure is illustrated in the region of a longitudinal end of the stent extending in the plane of the drawing. The primary form of this supporting structure generally produced in one piece by laser cutting from a tubular basic part is formed by three parallel helix webs 2.1, 2.2, 2.3 running helically in windings W about the longitudinal axis L of the cylindrical stent. Each of these helix webs 2.1, 2.2, 2.3 has a secondary form, which comprises meander bends 3.1, 3.2 with a direction of curvature alternating in the circumferential direction U, and also sub-webs 4.1, 4.2 connecting said meander bends 3.1, 3.2. The meander bends 3.1, 3.2 and sub-webs 4.1, 4.2 of this secondary form are produced here in an asymmetrical meander structure, in which each second sub-web 4.1 has a greater length than the sub-webs 4.2 arranged therebetween, as viewed in the circumferential direction U. The helix webs 2.1, 2.2, 2.3 therefore run in the manner of a thread having three turns with a pitch set by the difference in length between the sub-webs 4.1, 4.2 so as to form the circumferential wall 1 helically about the longitudinal axis L.

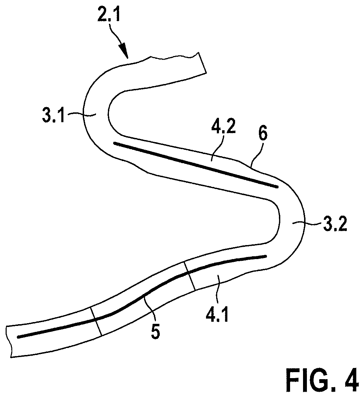

As can be seen from figure 4, the longer sub-webs 4.1 are curved inherently in a very flat S-shaped manner, which is indicated in this drawing by the line 5. The shorter sub-webs 4.2 are widened compared to the meander bends 3.1, 3.2 since outwardly protruding, flat ramps 6 are provided at the transition to these meander bends 3.1, 3.2.

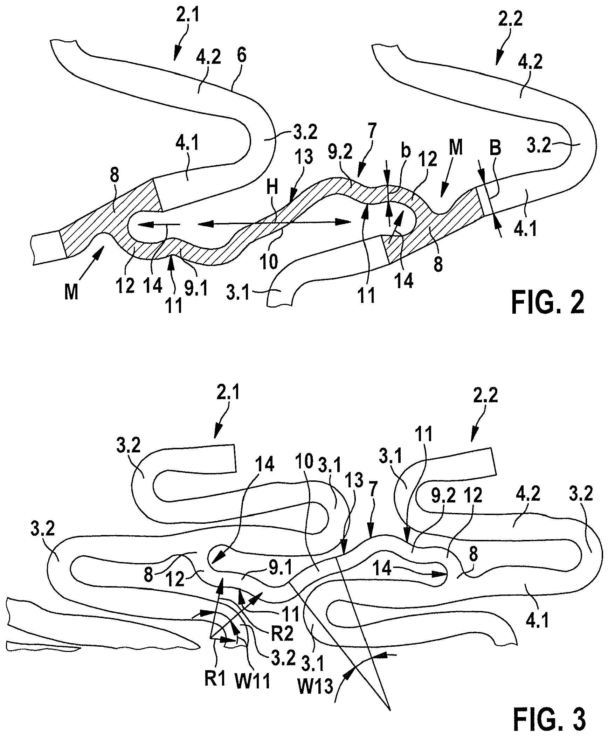

Whereas the sub-webs 4.1, 4.2 are oriented at an angle to the direction of the longitudinal axis L, for example at an angle from 5 to 40°, the three helix webs 2.1, 2.2, 2.3 are bridged in the region of two adjacent windings by connection webs 7, which run with their primary direction of extension H parallel to the longitudinal axis L of the stent structure. These connection webs 7 are each attached at their web-side ends 8 to the adjacent longer sub-webs 4.1 of the adjacent helix webs 2.1, 2.2 and 2.2, 2.3 and 2.3, 2.1 and have a flat, zigzag-like shape with two end branches 9.1, 9.2 and an intermediate central branch 10. As can be seen from figures 2 and 3, the two end branches 9.1, 9.2 have inherently a progression with a flat deflection bend 11 and, adjoining the sub-web 4.1, a termination bend 12, which has a direction of curvature opposite that of the respective deflection bend 11. Here, the radius R2 of the deflection bend 11 corresponds to approximately two to four times (in the drawing according to figure 3 specifically approximately three times) the radius R1 of the adjacent meander bend 3.1, 3.2. Both radii have a more or less common central point Z. The bend angle W11 of the deflection bend 11 is roughly approximately 30° in the shown exemplary embodiment. It is at most approximately 45°.

The central branch 10 has an undulating deflection bend 13, that is to say is inherently curved in a slightly S-shaped manner. The undulation here is so flat that the lateral spread of the two bends relative to one another does not go beyond the width b of the central branch 10. Due to the associated large radius of the deflection bends 13, the bend angle W13 thereof is likewise low and does not exceed 45°.

Lastly, the inner corners in the mouth region M between the termination bends 12 of the end branches 9.1, 9.2 and the respective sub-web 4.1 are provided with a recess 14. This leads to a reduction of the width B of the sub-webs 4.1 in the mouth region M by approximately 30 %. The dimensioning of the recess 14 can be selected on the whole such that width reductions in the range from 5 % to 50 %, preferably 20 % to 30 %, are produced.

As can be seen from figure 1, the connection webs 7 of two helix webs 2.1, 2.2 and 2.2, 2.3 and 2.3, 2.1 following successively in pairs are offset from one another in the circumferential direction such that, in each case, only one connection web 7 at most leads into a respective sub-web 4.1 of the helix webs 2.1, 2.2, 2.3.

The detailed view according to figure 2 shows the arrangement of the helix webs 2.1, 2.2 and of a connection web 7 of the stent in the state before crimping onto a catheter. The primary direction of extension H of the connection web 7 is therefore parallel to the longitudinal axis L of the stent.

The state of the stent before crimping onto a catheter is the state in which the production of the stent is concluded. In this state, it is applied ("crimped") onto the respective catheter in order to be introduced into the bodily vessel. There, it is then expanded to the respective diameter of the bodily vessel. The form in the expanded state, in particular the alignment of the individual webs and bends, then corresponds substantially to the state before crimping, wherein modifications due to the expansion and the adaptation to the bodily vessel are possible however.

In contrast to the arrangement and reciprocal position of the helix webs 2.1, 2.2 and of the connection web 7 relative to one another, the significance of the deflection bends 11 or double deflection bends 13 is in particular clear from figure 3. From this illustration, it is thus clear for example that the deflection bend 11 at the end branch 9.1 nestles around the meander bend 3.2 of the helix web 2.1, said meander bend being illustrated in the bottom left-hand corner in figure 3. The double deflection bend 13 of the central branch 10 is arranged with its sub-bend running in opposite directions in a nestling sweeping progression around the meander bend 3.1 of the helix web 2.2 illustrated in the lower right-hand corner and the meander bend 3.1 of the helix web 2.1 illustrated in the top, center region. The deflection bend 11 of the end branch 9.2 again nestles around the meander bend 3.1 of the helix web 2.2 illustrated in the upper right-hand corner. On the whole, the progression of the connection webs 7 between the helix webs 2.1, 2.2, 2.3 in the contracted state of the stent is thus optimized to such an extent that, due to the explained bend configurations, the space available between the webs in the contracted state is utilized optimally whilst meeting all demands that are placed on the behavior of the stent during crimping in the contracted state and during expansion.

Lastly, a first embodiment of the termination of the cylindrical circumferential wall at the longitudinal ends 15 thereof is to be explained with reference to figure 1. At both longitudinal ends 15, an edge segment 16 running around in the circumferential direction U of the circumferential wall 1 is thus provided and has a symmetrical meander structure with uniform end bends 17 and sub-webs 18 of equal length. In order to connect to the edge segment 16 the three helix webs 2.1, 2.2, 2.3 running diagonally into said edge segment 16 in the manner of a thread having three turns in order to form the circumferential wall 1, the circumferential helix webs 2.1, 2.2, 2.3 are to be attached in the embodiment shown in figure 1 to the respective inner end bend 17 of the edge segment 16 with increasing reduction of the length of their sub-webs 4.1, 4.2 as far as a coupling web 22.

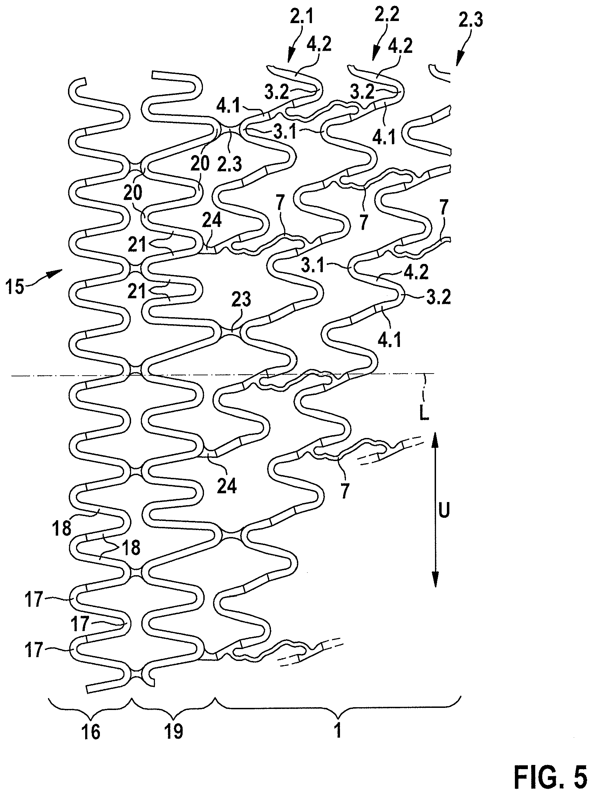

In the embodiment shown in figure 5, a transition segment 19 is provided between the discontinuing helix webs 2.1, 2.2, 2.3 and the edge segment 16, said transition segment being configured with matched radii of the meander bends 20 and suitably dimensioned length of the sub-webs 21. The helix webs 2.1, 2.2, 2.3 run in their pitch into the transition segment 19, without modification of their meander bends 3.1, 3.2 and sub-webs 4.1, 4.2, wherein, at a far projecting meander bend 20, the penultimate meander bend 3.1 pointing towards the longitudinal end 15 is attached to the suitably far projecting meander bend 20 of the transition segment 19 via a coupling web 23. At the end of each helix web 2.1, 2.2, 2.3, said helix web discontinues via a further short coupling web 24, running into a suitable adjacent meander bend 20, protruding less far inwardly, of the transition segment 19.

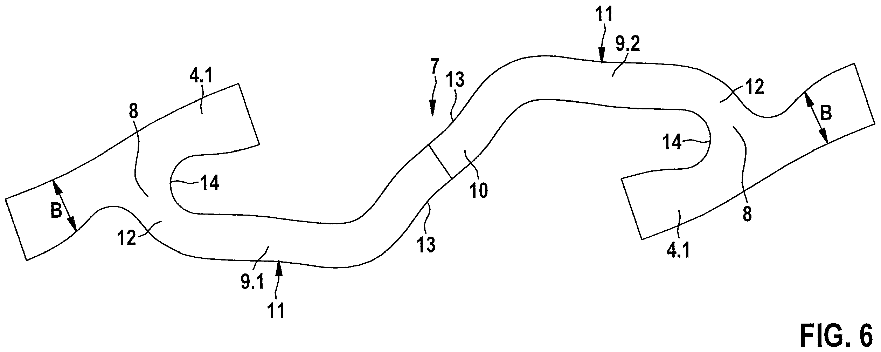

Figure 6 shows an embodiment of a stent according to the invention in the state before crimping, this embodiment being suitable in particular for use in very small bodily vessels. In this embodiment of the invention, the end branches 9.1, 9.2 and the central branch 10 are oriented at a much larger angle to one another than in the previous embodiments. The radius R2 of the deflection bends 11 to the radius R1 of the adjacent meander bend (not illustrated) forms a ratio of 11. The deflection bend 11 of the end branch 9.1, 9.2 spans a bend angle of approximately 9°, the deflection bend 13 of the central branch 10 spans a bend angle of approximately 23°. The radius R3 of the deflection bend 13 of the central branch to the radius R1 of the adjacent meander bend (not illustrated) forms a ratio of 2.3. Due to the recess 14 between the termination bend 12 and the meander part 4.1, the width of the part 4.1 is reduced by approximately 27 %.

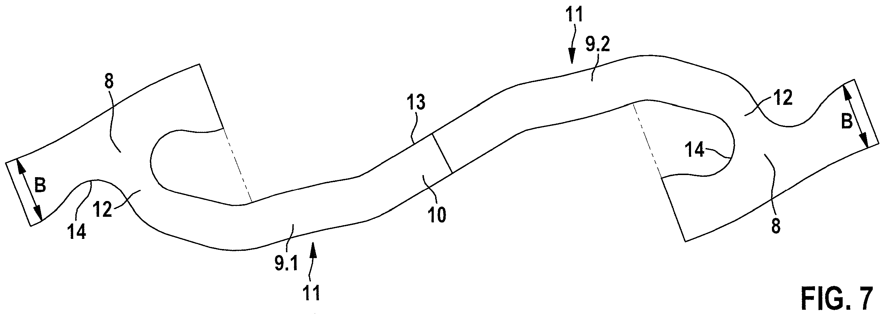

Figure 7 shows another alternative embodiment of the stent according to the invention in the state before crimping. In this embodiment, the end branches 9.1 and 9.2 have a much smaller angle to the central branch 10. The central branch 10 in this embodiment has an extremely flat undulating compensation bend 13, which is designed as a single deflection bend 13 and not as a double deflection bend 13. The radius R2 of the deflection bend 11 of the end branches 9.1 and 9.2 is 5 times the radius R1 of the adjacent meander bend (not illustrated). The radius R3 of the deflection bend 13 of the central branch to the radius R1 of the adjacent meander bend (not illustrated) forms a ratio of 11. Due to the recesses 14, the width B of the sub-webs 4.1 reduces by 27 %. A stent according to this embodiment of the invention has a greater rigidity in the axial direction than the embodiment illustrated in figure 6.

## Claims

1. A stent with a preferably cylindrical circumferential wall (1) designed as an open, lattice-like, expandable supporting structure with a longitudinal axis (L), said stent comprising
- helix webs (2.1, 2.2, 2.3) running around helically in windings (W) about the longitudinal axis (L) with a helical primary form and a meandering secondary form, which forms meander bends (3.1, 3.2) with a direction of curvature alternating in the circumferential direction (U) and also forms sub-webs (4.1, 4.2) connecting the meander bends (3.1, 3.2), and
- connection webs (7) bridging the helix webs (2.1, 2.2, 2.3) of two adjacent windings (W) with a primary direction of extension (H) parallel to the longitudinal axis (L), said connection webs being joined at their sub-web-side ends (8) to adjacent sub-webs (4.1) of the respective helix web (2.1, 2.2, 2.3) and having a flat, zigzag-like shape with two end branches (9.1, 9.2) and an intermediate central branch (10), the end branches (9.1, 9.2) each comprise a termination bend (12), each termination bend (12) forms with the respective sub-web (4.1) in a mouth region (M) right angled corners,
wherein the mouth region (M) is part of the sub-web (4.1) where the sub-web (4.1) and the termination bend (12) are joined, **characterised in that** each of the corners is provided with a recess (14), and
wherein the end branches (9.1, 9.2) each inherently have a deflection bend (11) formed flat in such a way that the radius (R2) thereof corresponds to 1 to 15 times the radius (R1) of an adjacent meander bend (3.1, 3.2) and the inner face of the deflection bends in the unexpanded state of the stent is arranged adjacently to the outer face of this meander bend (3.1, 3.2) of the secondary form of the helix webs (2.1, 2.2, 2.3) and wherein each termination bend (12) and the respective deflection bend (11) arranged therebefore of each connection web (7) are curved in opposite directions, wherein the deflection bends are webs in form of a bend, which is oriented in a direction around another structure to avoid contact with another structure.

2. The stent as claimed in claim 1, wherein due to the recess (14), the width (B) of the sub-webs (4.1) is reduced in the region of the recess (14) by 5 % to 50 %, preferably by 20 % to 30 %, particularly preferably by 24 % to 28 %.

3. The stent as claimed in claim wherein the radius (R2) of the flat deflection bend (11) corresponds to 1.3 to 12 times, preferably 4 to 11 times, particularly preferably 6 to 8 times, the radius (R1) of an adjacent meander bend (3.1, 3.2).

4. The stent as claimed in in one of the preceding claims, wherein the central branch (10) has an undulating deflection bend (13).

5. The stent as claimed in one of the preceding claims, wherein the deflection bends (11, 13) of the end branch and central branch (9.1, 9.2, 10) each span a bend angle (W11, W13) that is 45° at most.

6. The stent as claimed in one of the preceding claims, wherein three helix webs (2.1, 2.2, 2.3) run around helically about the longitudinal axis (L) in the manner of a thread having three turns so as to form the circumferential wall (L).

7. The stent as claimed in one of the preceding claims, wherein for dimensioning of the pitch of the helix webs (2.1, 2.2, 2.3), an asymmetrical meander structure is provided, in which each second (4.1) of the sub-webs (4.1, 4.2) of a helix web (2.1, 2.2, 2.3) following successively in the circumferential direction (U) has a greater length than the sub-webs (4.2) arranged therebetween.

8. The stent as claimed in claim 7, wherein the longer sub-webs (4.1) are each curved inherently in a S-shaped flat wave manner, wherein the flat wave has a ratio of wavelength to amplitude of at least 13:1.

9. The stent as claimed in one of the preceding claims, wherein a peripheral edge segment (16) with a meander structure designed in such a way that the supporting structure of the stent has a straight termination at right angles to its longitudinal axis (L) is arranged at the two longitudinal ends (15) of the circumferential wall (1).

10. The stent as claimed in claim 9, wherein the peripheral helix webs (2.1, 2.2, 2.3) run in a discontinuing manner into the edge segment (16) with increasing reduction of the length of their sub-webs (4.1, 4.2).

11. The stent as claimed in claim 10, wherein a transition segment (19) being configured with matched radii of its meander bends (20) and lengths of its sub-webs (21) is formed between the edge segment (16) and the helix webs (2.1, 2.2, 2.3) of the circumferential wall (1), the helix webs (2.1, 2.2, 2.3) running in a discontinuing manner into said transition segment, without reduction of the length of their sub-webs (4.1, 4.2).

## Patentansprüche

1. Stent mit bevorzugt zylindrischer umlaufender Wand (1), ausgelegt als offene, gitterartige, expandierbare Stützstruktur mit einer Längsachse (L), besagter Stent umfassend
- gewendelte Stränge (2.1, 2.2, 2.3), spiralförmig in Wicklungen (W) um die Längsachse (L) umlaufend, mit einer spiralförmigen Primärform und einer mäanderförmigen Sekundärform, die Mäanderbögen (3.1, 3.2) bildet, mit einer Krümmungsrichtung, die in umlaufender Richtung (U) alterniert, und auch Unterstränge (4.1, 4.2) bildet, die die Mäanderbögen (3.1, 3.2) verbinden, und
- Verbindungsstränge (7), die die gewendelten Stränge (2.1, 2.2, 2.3) zweier benachbarter Wicklungen (W) brückenförmig verbinden, mit einer primären Ausdehnungsrichtung (H) parallel zur Längsachse (L), wobei besagte Verbindungsstränge an ihren unterstrangseitigen Enden (8) an benachbarte Unterstränge (4.1) des jeweiligen gewendelten Strangs (2.1, 2.2, 2.3) gefügt sind und eine flache, zickzackförmige Form mit zwei Endabschnitten (9.1, 9.2) und einem mittigen Zwischenabschnitt (10) aufweisen, wobei die Endabschnitte (9.1, 9.2) jeweils eine Abschlussbiegung (12) umfassen, wobei jede Abschlussbiegung (12) mit dem jeweiligen Unterstrang (4.1) in einem Mündungsbereich (M) rechtwinklige Ecken bildet, wobei der Mündungsbereich (M) Teil des Unterstrangs (4.1) ist, wo der Unterstrang (4.1) und die Abschlussbiegung (12) zusammengefügt sind, **dadurch gekennzeichnet, dass** jede der Ecken einen Rücksprung (14) aufweist, und wobei die Endabschnitte (9.1, 9.2) jeweils inhärent eine Umlenkbiegung (11) aufweisen, die flach in einer Weise ausgebildet ist, dass der Radius (R2) derselben 1- bis 15-mal dem Radius (R1) eines benachbarten Mäanderbogens (3.1, 3.2) entspricht und die Innenfläche der Umlenkbiegungen im nicht expandierten Zustand des Stents benachbart zur Außenfläche dieses Mäanderbogens (3.1, 3.2) der Sekundärform der gewendelten Stränge (2.1, 2.2, 2.3) angeordnet ist und wobei jede Abschlussbiegung (12) und die jeweilige davor liegende Umlenkbiegung (11) jedes Verbindungsstrangs (7) in entgegengesetzte Richtungen gekrümmt sind, wobei die Umlenkbiegungen Stränge in Form einer Biegung sind, die in einer Richtung um eine andere Struktur ausgerichtet ist, um Kontakt mit einer anderen Struktur zu vermeiden.

2. Stent nach Anspruch 1, wobei aufgrund des Rücksprungs (14) die Breite (B) der Unterstränge (4.1) in der Region des Rücksprungs (14) um 5 % bis 50 %, bevorzugt um 20 % bis 30 %, besonders bevorzugt um 24 % bis 28 % reduziert ist.

3. Stent nach Anspruch 2, wobei der Radius (R2) der flachen Umlenkbiegung (11) 1,3-bis 12-mal, bevorzugt 4- bis 11-mal, besonders bevorzugt 6- bis 8-mal dem Radius (R1) eines benachbarten Mäanderbogens (3.1, 3.2) entspricht.

4. Stent nach einem der vorstehenden Ansprüche, wobei der mittige Abschnitt (10) eine wellenförmige Umlenkbiegung (13) aufweist.

5. Stent nach einem der vorstehenden Ansprüche, wobei die Umlenkbiegungen (11, 13) des Endabschnitts und mittigen Abschnitts (9.1, 9.2) jeweils einen Biegungswinkel (W11, W13) überspannen, der höchstens 45° beträgt.

6. Stent nach einem der vorstehenden Ansprüche, wobei drei gewendelte Stränge (2.1, 2.2, 2.3) in der Art eines dreimal umwickelten Fadens spiralförmig um die Längsachse (L) verlaufen, um die umlaufende Wand (1) zu bilden.

7. Stent nach einem der vorstehenden Ansprüche, wobei zur Dimensionierung der Steigung der gewendelten Stränge (2.1, 2.2, 2.3) eine asymmetrische Mäanderstruktur vorgesehen ist, bei der jeder zweite (4.1) der Unterstränge (4.1, 4.2) eines gewendelten Strangs (2.1, 2.2, 2.3), die in umlaufender Richtung (U) sukzessive aufeinanderfolgen, eine größere Länge als die dazwischen angeordneten Unterstränge (4.2) hat.

8. Stent nach Anspruch 7, wobei die längeren Unterstränge (4.1) jeweils inhärent in einer S-förmigen, flachwelligen Weise gekrümmt sind, wobei die flache Welle ein Verhältnis von Wellenlänge zu Amplitude von mindestens 13:1 aufweist.

9. Stent nach einem der vorstehenden Ansprüche, wobei ein peripheres Kantensegment (16) mit einer Mäanderstruktur, die so ausgelegt ist, dass die Stützstruktur des Stents einen geraden Abschluss in rechten Winkeln zu ihrer Längsachse (L) hat, an den zwei Längsenden (15) der umlaufenden Wand (1) angeordnet ist.

10. Stent nach Anspruch 9, wobei die peripheren gewendelten Stränge (2.1, 2.2, 2.3) auf diskontinuierliche Art in das Kantensegment (16) laufen, mit fortschreitender Reduzierung der Länge ihrer Unterstränge (4.1, 4.2).

11. Stent nach Anspruch 10, wobei ein Übergangssegment (19), das mit passenden Radien seiner Mäanderbögen (20) und Längen seiner Unterstränge (21) ausgelegt ist, zwischen dem Kantensegment (16) und den gewendelten Strängen (2.1, 2.2, 2.3) der umlaufenden Wand (1) ausgebildet ist, wobei die gewendelten Stränge (2.1, 2.2, 2.3) auf diskontinuierliche Art in besagtes Übergangssegment laufen, ohne Reduzierung der Länge ihrer Unterstränge (4.1, 4.2).

## Revendications

1. Endoprothèse pourvue d'une paroi (1) circonférentielle de préférence cylindrique conçue sous forme d'une structure de support ouverte, semblable à un réseau expansible avec un axe longitudinal (L), ladite endoprothèse comprenant :
- des bandes en hélices (2.1, 2.2, 2.3) décrivant de manière hélicoïdale des enroulements (W) autour d'un axe longitudinal (L) avec une forme en hélice primaire et une forme en méandres secondaire, qui forme des coudes en méandres (3.1, 3.2) avec une direction de courbure alternant dans la direction circonférentielle (U) et forme également des bandes secondaires (4.1, 4.2) reliant les coudes en méandres (3.1, 3.2), et
- des bandes de connexion (7) formant des ponts avec les bandes en hélices (2.1, 2.2, 2.3) de deux enroulements (W) adjacents avec une direction primaire d'extension (H) parallèle à l'axe longitudinal (L), lesdites bandes de connexion étant jointes à leurs extrémités du côté bande secondaire (8) à des bandes secondaires (4) adjacentes de la bande en hélice (2.1, 2.2, 2.3) respective et ayant une silhouette plate, semblable à un zigzag, avec deux branches d'extrémité (9.1, 9.2) et une branche centrale intermédiaire (10), les branches d'extrémité (9.1, 9.2) comprennent chacune un coude de terminaison (12), chaque coude de terminaison (12) forme avec la bande secondaire (4.1) respective des coins à angles droits dans une région d'embouchure (M),
dans laquelle la région d'embouchure (M) fait partie de la bande secondaire (4.1) à l'endroit où la bande secondaire (4.1) et le coude d'extrémité (12) sont joints, **caractérisée en ce que** chacun des coins est pourvu d'un renfoncement (14) et dans laquelle les branches d'extrémités (9.1, 9.2) ont chacune de manière inhérente un coude de déviation (11) formé à plat dans une manière telle que son rayon (R2) corresponde à 1 à 15 fois le rayon (R1) d'un coude en méandres (3.1, 3.2) adjacent et la face intérieure des coudes de déviation dans l'état non expansé de l'endoprothèse est disposée de manière adjacente par rapport à la face extérieure de ce coude en méandres (3.1, 3.2) de la forme secondaire des bandes en hélices (2.1, 2.2, 2.3) et dans laquelle chaque coude de terminaison (12) et le coude de déviation (11) respectif sont disposés avant que chaque bande de connexion (7) ne soit courbée dans des directions opposées, dans laquelle les coudes de déviation sont des bandes sous forme d'un coude qui est orienté dans une direction autour d'une autre structure afin d'éviter un contact avec l'autre structure.

2. Endoprothèse selon la revendication 1, dans laquelle, à cause du renfoncement (14), la largeur (B) des bandes secondaires (4.1) est réduite dans la région du renfoncement (14) de 5 % à 50 %, de préférence de 20 % à 30 %, de manière particulièrement préférée de 24 % à 28 %.

3. Endoprothèse selon la revendication 1, dans laquelle le rayon (R2) du coude de déviation (11) plat correspond à 1,3 à 12 fois, de préférence de 4 à 11 fois, de manière particulièrement préférée de 6 à 8 fois, le rayon (R1) d'un coude en méandres (3.1, 3.2) adjacent.

4. Endoprothèse selon l'une des revendications précédentes, dans laquelle la branche centrale (10) a un coude de déviation (13) ondulant.

5. Endoprothèse selon l'une des revendications précédentes, dans laquelle les coudes de déviation (11, 13) de la branche d'extrémité et de la branche centrale (9.1, 9.2, 10) forment chaque fois un angle de courbure (W11, W13) qui fait au plus 45 °.

6. Endoprothèse selon l'une des revendications précédentes, dans laquelle trois bandes en hélice (2.1, 2.2, 2.3) tournent de manière hélicoïdale autour de l'axe longitudinal (L) à la manière d'un fil ayant trois tours de sorte à former la paroi circonférentielle (L).

7. Endoprothèse selon l'une des revendications précédentes, dans laquelle, pour dimensionner le pas des bandes en hélices (2.1, 2.2, 2.3), on met en place une structure en méandres asymétrique dans laquelle chaque deuxième bande secondaire (4.1) parmi les bandes secondaires (4.1, 4.2) d'une bande en hélice (2.1, 2.2, 2.3) se succédant dans la direction circonférentielle (U) a une longueur plus grande que les bandes secondaires (4.2) disposées entre elles.

8. Endoprothèse selon la revendication 7, dans laquelle les bandes secondaires (4.1) plus longues sont courbées de manière inhérente à la manière d'une onde plate en forme de S, dans laquelle l'onde plate a un rapport longueur d'onde sur amplitude d'au moins 13 : 1.

9. Endoprothèse selon l'une des revendications précédentes, dans laquelle un segment de bord périphérique (16) avec une structure en méandres, conçu de telle manière que la structure de support de l'endoprothèse ait une terminaison droite aux angles droits par rapport à son axe longitudinal (L), est disposé aux deux extrémités longitudinales (15) de la paroi circonférentielle (1).

10. Endoprothèse selon la revendication 9, dans laquelle les bandes en hélices (2.1, 2.2, 2.3) périphériques s'étendent de manière discontinue dans le segment de bord (16) avec une réduction de la longueur de leurs bandes secondaires (4.1, 4.2) croissante.

11. Endoprothèse selon la revendication 10, dans laquelle un segment de transition (19) conçu avec des rayons de ses coudes en méandres (20) et des longueurs de ses bandes secondaires (21) concordants est formé entre le segment de bord (16) et les bandes en hélices (2.1, 2.2, 2.3) de la paroi circonférentielle (1), les bandes en hélices (2.1, 2.2, 2.3) s'étendant de manière discontinue dans ledit segment de transition, sans réduction de la longueur de leurs bandes secondaires (4.1, 4.2).
